Europäisches Patentamt

European Patent Office

Office européen des brevets

(19)

(11) Numéro de publication : **0 584 023 A1**

# (12) DEMANDE DE BREVET EUROPEEN

(21) Numéro de dépôt : 93420339.9

(22) Date de dépôt : 12.08.93

(51) Int. Cl.⁵ : **C12N 15/31,** C12Q 1/68, A61K 39/04

Une requête en rectification a été présentée conformément à la règle 88 CBE. Il est statué sur cette requête au cours de la procédure engagée devant la division d'examen (Directives relatives à l'examen pratiqué à l'OEB, A-V, 2.2).

Le demandeur a ultérieurement déposé une liste des séquences et déclaré, que cette liste ne comporte aucun élément nouveau.

(30) Priorité : **12.08.92 FR 9210094**

(43) Date de publication de la demande :
**23.02.94 Bulletin 94/08**

(84) Etats contractants désignés :
**BE CH DE ES GB IT LI NL**

(71) Demandeur : **BIO MERIEUX**
**F-69280 Marcy l'Etoile (FR)**

(72) Inventeur : **Mabilat, Claude**
**1 Cours Emile Zola**
**F-69100 Villeurbanne (FR)**
Inventeur : **Pechere, Jean-Claude**
**4 rue de l'Athénie**
**CH-1205 Geneve (CH)**

(74) Mandataire : **Maureau, Philippe et al**
**Cabinet GERMAIN & MAUREAU BP 3011**
**F-69392 Lyon Cédex 03 (FR)**

(54) **Fragments d'ADN de mycobactéries, amorces d'amplification, sondes d'hybridation, réactifs et procédé de détection de mycobactéries.**

(57) Fragment nucléotidique d'ADN, dont la séquence nucléotidique est incluse dans le gène des espèces du genre Mycobacterium, codant pour l'antigène mycobactérien 65 kD, comportant des régions homologues à pratiquement toutes les espèces du genre Mycobacterium, et au moins une région variable spécifique à l'espèce, **caractérisé en ce que** ledit fragment est choisi parmi les fragments dont les séquences nucléotidiques présentent au moins 70 % d'homologie, et préférentiellement au moins 85 % d'homologie, avec une séquence prédéterminée ou sa séquence complémentaire, ladite séquence prédéterminée commençant au nucléotide 438 et finissant au nucléotide 751 dudit gène codant pour ledit antigène de toutes les espèces de mycobactéries à l'exclusion des espèces *M. tuberculosis*, *M. bovis BCG*, *M. avium*, *M. paratuberculosis*, *M. fortuitum*, *M. malmoense*, *M. laprea*, *M. kansaii* et *M. marinum*.

Figure 1a

EP 0 584 023 A1

Figure 1b

Figure 1c

578
```
tuberculosis (1) CGCCAACCCG CTCGGTCTCA AACGCGGCAT CGAAAAGGCC GTGGAGAAGG TCACCGAGAC CCTGCTCAAG
bovis BCG   (2) ---------- ---------- ---------- ---------- ---------- ---------- ----------
bovis       (3) ---------- ---------- ---------- ---------- ---------- ---------- ----------
microti     (4) ---------- ---------- ---------- ---------- ---------- ---------- ----------
africanum   (5) ---------- ---------- ---------- ---------- ---------- ---------- ----------
chitae      (6) -----C--G- ---------- -G-------- ----C--CC- ---------- ---T-G---A ----------
intracel.3324(7) --G-----G- ---------- -G-------- ----C---C- ---------- ---------- ----------
intracellulare(8) --G----A-- ---------- -G-------- ----C----- ---------- ---------- ----------
avium       (9) --G------- --G------- -G-------- ----C----- ---------- ---------- ----------
paratuberculosis(10) -G-------- ---C--G- -G-------- ----C----- ---------- ---------- -G------G-
fortuitum   (11) ---------- --A-C---- -G-------- ----C----- ---------- ---------- -G------G-
malmoense   (12) --T------- --GA-C--- -G-------G ----C----- ---------- ---------- ----G-----
scrofulaceum(13) ---------- ---------- -G-------G ----C----- ---------- ---------- ----G-----
leprae      (14) --A------- --A------- -C--T----- --G---CTG --C--T---- -A--T----- -T--------
```

648
```
tuberculosis (1) GGCGCCAAGG AGGTCGAGAC CAAGGAGCAG ATTGCGGCCA CCGCAGCGAT TTCGGCGGGT GACCAGTCCA
bovis BCG   (2) ---------- ---------- ---------- ---------- ---------- ---------- ----------
bovis       (3) ---------- ---------- ---------- ---------- ---------- ---------- ----------
microti     (4) ---------- ---------- ---------- ---------- ---------- ---------- ----------
africanum   (5) ---------- ---------- ---------- ---------- ---------- ---------- ----------
chitae      (6) --TCG----- ------C--- -------C-- --C-C----- --C-G----- ---C---C-- --A-CA----
intracel.3324(7) --TCG----A ------C--- -------C-- --C-T----- --G--C---- ---C----C -------C--
intracellulare(8) --TCG----- ------C--- -------C-- --C-T----- --G--C---- --G------C -----CGG--
avium       (9) --TCG----- ---------- -------C-- --C-T----- ---G--C--- --C--C---C ------G---
paratuberculosis(10) --TCG----- ---------- -------C-- --C-T----- --G--C---- --C-C--C-- ------G---
fortuitum   (11) --A------- ---G----- -------C-- --C-T--G- --C-T-G--- --C-C-C--- -------G--
malmoense   (12) --TCG----- ---------- -------C-- --C-C--G- --C-C--C-- -----C---- -----G----
scrofulaceum(13) --TCG----- ---------- ---------- ------T--- --T------- --------G- -----C----
leprae      (14) --A-A----- -----A--- ---A--A----- -----T--- ----------- --T------- ------G---
```

718
```
tuberculosis (1) TCGGTGACCT GATCGCCGAG GCGGATGGACA AGGTGGGCCAA CGAGGGCGTC ATCACCGTCG AGGAGTCCAA
bovis BCG   (2) ---------- ---------- ---------- ---------- ---------- ---------- ----------
bovis       (3) ---------- ---------- ---------- ---------- ---------- ---------- ----------
microti     (4) ---------- ---------- ---------- ---------- ---------- ---------- ----------
africanum   (5) ---------- ---------- ---------- ---------- ---------- ---------- ----------
chitae      (6) ---------- ---------- ---------- ---------- ---------- ---------- ----------
intracel.3324(7) ---------C ---------- ---------- ---------- ---------- ---------- ----------
intracellulare(8) ---------- ---------- ---------- ---------- ---------- ---------- ----------
avium       (9) -----C---- ---------- ---------- ---------- ---------- ---------- ----------
paratuberculosis(10) ---C---- ---------- ---------- ---------- ---------- ---------- ----------
fortuitum   (11) ---------- ----C----- ----------- ----------- ---------- ---------- ----------
malmoense   (12) -----C---- ----C----- ----------- ----------- ---------- ---------- ----------
scrofulaceum(13) -------T-- ----C----- ----------- ----------- ---------- ---------- ----------
leprae      (14) ---------- ----T----- ----------- ----------- ---------- ---------- ----------
```

2

Le genre *Mycobacterium* inclut au moins 54 espèces (Wayne, L.G., and Kubica, G.P. 1986. Genus *Mycobacterium.* In "Bergey's Manual of Systematic Bacteriology" (P.H.A. Sneath, N. Mair, and M.E. Sharp, eds.). Vol 2, pp. 1436-1457. Williams & Wilkins, Baltimore, Maryland). La plupart de ces espèces sont saprophytes et ne causent pas de maladies humaines ou vétérinaires. Les mycobactéries pathogènes pour l'homme les plus importantes en terme de morbidité et de mortalité sont *Mycobacterium tuberculosis* et *M. leprae*, qui causent la tuberculose et la lèpre. La tuberculose reste une des grandes maladies infectieuses de la planète, avec quelques 10 millions de nouveaux cas et 3 millions de décès par année (Stylbo, K. 1983. Tuberculosis and its control: Lessons to be learned from past experience and its implications for leprosy control programmes. Ethiop. Med. J. 21:101-122. et World Health Organisation.1986. Results of a World Health Organisation-sponsored workshop to characterize antigens recognized by *Mycobacterium*-specific monoclonal antibodies. Infect. Immun. 51:718-720).

En Europe, en Afrique, en Amérique du Nord, une tendance récente à une augmentation de prévalence semble se dessiner, sans doute liée à la multiplication des cas de SIDA (Stead, W.W., and Dutt, A.K. 1988. Changing faces of clinical tuberculosis. In *"Mycobacterium tuberculosis.* Interactions with the Immune System" (M. Bendinelli and H. Friedman, eds.) pp.371-388. Plenum, New York.)

*M. tuberculosis* est taxonomiquement très proche de *M. bovis, M. africanum* (qui causent aussi des tuberculoses chez l'homme) et *M. microti* (tuberculose de certains rongeurs), de telle sorte qu'on réunit ces quatre espèces sous le nom de "complexe *M. tuberculosis*" (Wayne LG et al.). Parmi les mycobactéries non tuberculeuses (parfois appelées "atypiques"), il faut mentionner l'incidence accrue des mycobactéries appartenant au complexe *M. avium- intracellulare* chez des malades immunodéprimés (SIDA, transplantations, traitements du cancer...) (Kielin, T.E., Edwards, F.F., Brannon, P., Tsang, A. Y., Maio, M., Gold, J.W.M.,Whimby, E., Wong, B., McClatchy, J.K., and Amstrong, D. 1985. Infections caused by *Mycobacterium avium* complex in immunocompromised patients: Diagnosis by blood culture and fecal examination, antimicrobial susceptibility tests and morphological and seroagglutination characteristics. J. Clin. Microbiol. 21, 168-173. Macher, A.M., M. Kovacs, J.A., Gill, V., Roberts, G.D., Ames, J., Parke, C.H., Strans, S., Lane, H.C., Parillo, J.E., Fanci, A.S., and Masur, H. 1983. Bacteremia due to *Mycobacterium avium-intracellulare* in the acquired d'immunodeficiency syndrome. Ann. Intern. Med. 99, 782-785.)

Ce complexe comprend les espèces *M. avium, M. intracellulare* (très proches l'une de l'autre, d'où le terme *M. avium-intracellulare), M. paratuberculosis* (cause de la maladie de Johne chez le veau) et *M. lepraemurium* (lèpre du rat). D'autres espèces produisent des infections humaines de moindre importance en terme de gravité ou de morbidité, comme *M. kansasii* (adénites), *M. ulcerans* et *M. marinum* (ulcérations cutanées).

La quasi-totalité des mycobactéries présentent un antigène caractéristique et spécifique, dit antigène 65 kD, complètement séquencé et identifié.

L'antigène 65 kD présente nombre de caractéristiques intéressantes qui ont conduit à des études extensives. Tout d'abord cette protéine apparaît comme un antigène mycobactérien majeur. Les individus ou les animaux infectés ou immunisés avec des mycobactéries produisent des anticorps et des cellules-T qui reconnaissent cet antigène dans la grande majorité des cas, ce qui a permis d'ailleurs d'en disséquer les épitopes. Ensuite l'antigène 65 kD fait partie de la famille des protéines "heat-shock" ou protéines de choc thermique, également retrouvées avec un grand degré de conservation dans des nombreuses cellules procaryotes et eucaryotes. Ces protéines fonctionnent comme "chaperon" dans l'assemblage post-traductionnel de certaines protéines de procaryotes, chloroplastes et mitochondries (Ellis J. 1988. Nature 328: 378-9). Enfin, un intérêt particulier est aussi porté à l'antigène 65 kD dans la mesure où il a été associé à la pathogénèse d'arthrites auto-immunes (Thole, J.E.R., and Van Der Zee, R. 1990. The 65 kD antigen: molecular studies on a ubiquitous antigen. In: Molecular Biology of the Mycobacteria, J. Mc Fadden Ed., Surrey University Press, London, pp. 37-67).

Ces points d'intérêt multiples expliquent pourquoi cet antigène a été l'un des tout premier a être cloné et séquencé chez diverses mycobactéries (Clark-Curtiss, J.E., Jacobs, W.R., Docherty, M.A., Richtie, L.R., and Curtiss III, R. 1985. J. Bacteriol. 161, 1093-102. Young R.A., Blooms, B.R., Grosskinsky, C.M., Ivangi, J., Thomas, D.and Davis R.W. 1985. Proc. Natl. Acad. Sci. USA. 42:2583-7 Young, R.A., Mehra, V., Sweeetser, D., Buchanan, T, Clark-Curtiss, J., Davis, R.W., and Bloom, B.R. 1985. Genes for the major protein antigens of the leprosy parasite *Mycobacterium leprae.* Nature. 316:450-2. Lu, M.C., Lien, M.H., Becken, R.E., Heine, H.C., Buggo, A.M., Lipovsek, D., et al. 1987. Infect. Imm. 55:23-82. Husson R.N., and Younf R.A.. 1987. Proc. Natl. Acad. Sci. USA. 84:1679-83. Andersen, A.S., Worsaae, A, and Chaparas, S.D. 1988. Infect. Imm. 56:1344-51. Shinnick, T.M. 1987. The 65-kilodalton antigen of *Mycobacterium tuberculosis.* J. Bacteriol. 169:1080-88. Thole, J.E.R., Dauwesse, H.G., Das, P.K., Croothuis, D.G., Shouls, L.M. and Embden, J.D.A. 1985. Cloning of *Mycobacterium bovis* BCG DNA and expression of antigens in *Escherichia coli.* Inf. Imm. 50:800-6. Mehra, V., Sweetser D., and Young, R.A. 1986. Efficient mapping of protein antigenic determinants. Proc. Natl. Acad. Sci. USA. 83:7013-17.).

Le document WO-A-8800974, repris dans la référence de Young RA et al., Nature, 1985, 376:450-2, décrit une séquence d'ADN codant pour l'antigène 65 kD de *M. leprae,* le document WO-A-8806591 décrit une séquence d'ADN codant pour l'antigène 65 kD de *M. tuberculosis* reprise dans la référence Shinnick et al, et la publication de Thole et al, Infect. Immunol., 1987, 55:1466-71 décrit une séquence d'ADN codant pour l'antigène 65 kD de M. bovis BCG.

Cependant, toutes les techniques d'identification ou caractérisation qualitative et/ou quantitative de mycobactéries, décrites par l'art antérieur, directement à partir de leurs acides nucléiques, présentent encore les inconvénients suivants.

La séquence d'ADN génomique, retenue aux fins d'identifier des zones quasi-constantes (à la notion d'homologie près) au genre Mycobacterium, et/ou des zones variables respectivement spécifiques aux espèces appartenant audit genre, n'est pas conservée chez la plupart des mycobactéries connues à ce jour. Elle n'est en général conservée que pour quelques espèces, ce qui rend le processus d'identification non sélectif pour la totalité du genre Mycobacterium.

Plus récemment, le document WO-A-9012875 a décrit une séquence nucléotidique de 383 paires de bases du gène codant pour l'antigène 65 kD chez *M. tuberculosis, M. bovis BCG, M. avium, M. paratuberculosis, M. fortuitum, M. malmoense, M. leprae, M. kansaii* et *M. marinum,* séquence à partir de laquelle des sondes pour l'identification de certaines des espèces précitées ou groupes d'espèces ont été déterminées; ainsi que des amorces pour l'amplification de fragments d'ADN appartenant audit gène.

Néanmoins, les zones retenues par l'art antérieur ne sont conservées que pour quelques espèces, de sorte que la ou les amorces déterminées pour amplifier ces zones n'hybrident pas l'ADN génomique de certaines espèces, comme le mettent en évidence les résultats obtenus avec les amorces TB1 et TB2 selon WO-A-90 12875 et montrés dans le tableau 2.

La présente invention a pour objet de remédier aux inconvénients précédents. Plus précisément, l'invention a pour objet :

1) une séquence de l'ADN génomique des mycobactéries, appartenant au gène codant pour l'antigène mycobactérien 65 kd, comprenant des régions quasi-constantes pour la majeure partie des espèces de mycobactéries ;

2) toute amorce spécifique pour l'amplification par polymérisation d'ADN, de la séquence selon (1), quasi-constante pour la majeure partie des espèces appartenant au complexe *M tuberculosis* et/ou *M avium-intracellulare* ;

3) une ou des sondes de détection, dites sondes de genre, s'hybridant sur une partie de la séquence selon (1), quasi-constante pour la majeure partie des espèces appartenant au genre *Mycobacterium* ;

4) une ou plusieurs sondes de détection, dites sondes d'espèces, s'hybridant sur une partie de la séquence selon (1), quasi-constante pour la majeure partie des espèces appartenant au complexe *M tuberculosis* et/ou *M avium-intracellulare* ;

5) tout réactif ou réactifs mettant en jeu une ou plusieurs séquences isolées selon (1), et/ou des amorces selon (2), et/ou des sondes de genre selon (3), et/ou des sondes d'espèces selon (4).

Les termes sondes et/ou amorces tels qu'utilisés dans la présente invention font référence à un fragment d'ADN ou d'ARN naturel, ou un oligonucléotide naturel ou de synthèse, ou un fragment d'ADN ou d'ARN de synthèse non modifié ou comprenant une ou plusieurs bases modifiées telles que l'inosine, la méthyl-5-désoxycytidine, la désoxyuridine, la diméthylamino-5-désoxyuridine, la diamino-2,6-purine, la bromo-5-désoxyuridine ou toute autre base modifiée permettant l'hybridation, le fragment d'ADN pouvant être simple ou double brin.

Selon la présente invention, on propose tout d'abord un fragment monocaténaire d'ADN, isolé ou faisant partie d'une macro-molécule d'ADN bicaténaire, dont la séquence nucléotidique est incluse dans le gène des espèces du genre *Mycobacterium,* codant pour l'antigène mycobactérien dit 65 kD, comportant des régions constantes ou homologues à pratiquement toutes les espèces du genre mycobactéries, et une ou plusieurs régions variables, spécifiques, c'est-à-dire constantes ou homologues pour une espèce donnée. Selon l'invention, et à partir du séquençage du gène précité établi par Shinnick (Schnnick TM 1987, J. Bacteriol. 169: 1080-88), ledit fragment est choisi parmi les fragments dont les séquences nucléotidiques présentent au moins 70 % d'homologie et préférentiellement au moins 85 % d'homologie, avec une séquence prédéterminée ou de référence, ou homologue avec la séquence complémentaire de cette séquence prédéterminée ou de référence choisie selon l'invention, ladite séquence prédéterminée commençant au nucléotide 438 et finissant au nucléotide 751 dudit gène codant pour ledit antigène 65 kD de toutes les espèces de mycobactéries à l'exclusion des espèces *M. tuberculosis, M. bovis BCG, M. avium, M. paratuberculosis, M. fortuitum, M. malmoense, M. leprae, M. kansaii* et *M. marinum.*

Avant d'exposer l'invention, différents termes utilisés dans la description et les revendications sont à présent définis :

- selon l'invention, un fragment nucléotidique est un enchaînement de monomères, susceptibles de s'hybrider à un fragment nucléotidique dans des conditions prédéterminées, l'enchaînement pouvant contenir des monomères de structures différentes et être obtenu à partir d'une molécule d'acide nucléique naturelle et/ou par recombinaison génétique et/ou par synthèse chimique,
- ainsi un monomère peut être un nucléotide naturel d'acide nucléique dont les éléments constitutifs sont un sucre, un groupement phosphate et une base azotée; dans l'ADN le sucre est le ribose, dans l'ARN le sucre est le désoxy-2-ribose; selon qu'il s'agisse de l'ADN ou l'ARN, la base azotée est choisie parmi l'adénine, la guanine, l'uracile, la cytosine, la thymine; ou un nucléotide modifié dans l'un au moins des trois éléments constitutifs; à titre d'exemple, la modification peut intervenir au niveau des bases, générant des bases modifiées telles que l'inosine, la méthyl-5-désoxycytidine, la désoxyuridine, la diméthylamino-5-désoxyuridine, la diamino-2,6-purine, la bromo-5-désoxyuridine et toute autre base modifiée favorisant l'hybridation, au niveau du sucre à savoir le remplacement d'au moins un désoxyribose par un polyamide (P.E. Nielsen et al, Science, 254, 1497-1500 (1991)), au niveau du groupement phosphate par exemple son remplacement par des esters notamment choisis parmi les esters de diphosphate, d'alkyl et arylphosphonate et de phosphorothioate,
- par séquence complémentaire, on entend toute séquence s'hybridant totalement avec la séquence prédéterminée ou de référence,
- par hybridation, on entend le processus au cours duquel, dans des conditions appropriées, deux fragments nucléotidiques, ayant des séquences suffisamment complémentaires s'apparient pour former un double brin,
- une sonde est un fragment nucléotidique comprenant de 5 à 100 monomères, avantageusement de 10 à 40 monomères, possédant une spécificité d'hybridation dans des conditions déterminées pour former un complexe d'hybridation avec un fragment nucléotidique ayant une séquence nucléotidique comprise dans l'ADN génomique des mycobactéries; une sonde peut être utilisée à des fins de diagnostic telles que les sondes de capture et/ou de détection,
- la sonde de capture peut être immobilisée sur un support solide par tout moyen approprié, c'est-à-dire directement ou indirectement, par exemple par covalence ou adsorption passive,
- la sonde de détection est marquée au moyen d'un marqueur choisi parmi les isotopes radioactifs, des enzymes notamment choisis parmi la péroxydase et la phosphatase alcaline et ceux susceptibles d'hydrolyser un substrat chromogène, fluorigène ou luminescent, des composés chimiques chromophores, des composés chromogènes, fluorigènes ou luminescents, des analogues de bases nucléotidiques, et la biotine,
- les sondes utilisées à des fins de diagnostic de l'invention peuvent être mises en oeuvre dans toutes les techniques d'hybridation connues et notamment les techniques dites "DOT-BLOT" (MANIATIS et al, Molecular Cloning, Cold Spring Harbor, 1982), "SOUTHERN BLOT" (SOUTHERN. E.M., J. Mol. Biol., 98, 503 (1975), "NORTHEN BLOT" qui est une technique identique à la technique "SOUTHERN BLOT" mais qui utilise de l'ARN comme cible, la technique SANDWICH (DUNN A.R., HASSEL J.A., Cell, 12, 23 (1977)); avantageusement, on utilise la technique SANDWICH dans la présente invention comprenant une sonde de capture spécifique et/ou une sonde de détection spécifique, étant entendu que la sonde de capture et la sonde de détection doivent présenter une séquence nucléotidique au moins partiellement différente,
- une amorce est une sonde comprenant de 5 à 30 monomères, et de préférence 15 à 25 monomères, possédant une spécificité d'hybridation dans des conditions déterminées pour l'initiation d'une polymérisation enzymatique par exemple dans une technique d'amplification telle que la PCR (Polymerase Chain Reaction), dans un procédé d'élongation, tel que le séquençage, dans une méthode de transcription ou analogue,
- l'homologie caractérise le degré de similitude de deux fragments nucléotidiques comparés.

A titre d'exemple de fragments selon l'invention, la séquence du fragment monocaténaire présente au moins 70 % d'homologie, et préférentiellement au moins 85 % d'homologie, avec l'une quelconque des séquences SEQ ID N01, SEQ ID N02, SEQ ID N03, SEQ ID N04, SEQ ID N05 et SEQ ID N07, identifiées en fin de description.

Grâce au fragment monocaténaire selon l'invention, amplifié avec toutes amorces appropriées, et détecté avec toutes sondes de genre et d'espèces déterminées ci-après, toutes les mycobactéries testées, soit 21 souches représentant 19 espèces, ont répondu positivement au test effectué, là où des essais comparables, par exemple selon le document WO-A-90 12875, ne permettent d'amplifier que 15 souches sur 21, laissant par conséquent non détectées 6 souches de mycobactéries.

L'invention concerne également toute macromolécule d'ADN génomique ou isolé, ou d'ARN, comprenant ou intégrant un fragment monocaténaire tel que défini précédemment. Répondent à cette définition, notamment tout vecteur de réplication incorporant undit fragment, mais aussi tout produit d'amplification, résultant du marquage par des amorces appropriées d'une séquence nucléotidique correspondant à la séquence prédéterminée définie précédemment, à au moins 70% d'homologie près ; dans ce cas, on obtient une séquence nucléotidique dont la partie centrale correspond, sous forme monocaténaire, à la séquence prédéterminée, flanquée des deux amorces d'amplification par exemple.

A partir de la séquence prédéterminée retenue, l'invention a défini différentes amorces spécifiques pour l'amplification par polymérisation de l'ADN génomique d'une bactérie du genre mycobactérie. De manière générale, une telle amorce comprend une séquence nucléotidique lui permettant d'hybrider une zone dite constante de cette séquence prédéterminée, homologue ou identique pour pratiquement toutes les espèces du genre mycobactéries, notamment la zone commençant au nucléotide 438 et finissant au nucléotide 457, et la zone commençant au nucléotide 733 et finissant au nucléotide 751, toujours selon le séquençage de Shinnick.

Cette amorce comprend avantageusement entre 15 et 25 monomères.

A titre d'exemple, cette amorce présente une séquence nucléotidique choisie parmi les SEQ ID N08 et SEQ ID N09, identifiées en fin de description.

Toute technique d'amplification peut être utilisée, et en particulier on retiendra tous couples d'amorces tels que définis comme précédemment, par exemple tous couples d'amorces comprenant au moins 10 bases des séquences SEQ ID N08 et SEQ ID N09.

Toujours à partir du même fragment monocaténaire selon l'invention, cette dernière propose aussi une sonde dite de genre, susceptible d'hybrider une zone constante dudit fragment, homologue ou constante pour pratiquement toutes espèces du genre mycobactéries.

Préférentiellement, cette sonde de genre est susceptible d'hybrider une zone extrême du fragment monocaténaire selon l'invention, correspondant à une amorce telle que définie précédemment, intégrée ou liée audit fragment.

Mais, cette sonde de genre peut hybrider toute autre zone constante du même fragment monocaténaire ; avantageusement, ces sondes présentent une séquence nucléotidique choisi parmi les SEQ ID N010 et SEQ ID N011, identifiées en fin de description.

Toujours à partir du même fragment monocaténaire selon l'invention, d'autres objets de cette dernière sont différentes sondes d'espèce, comprenant chacune une séquence nucléotidique lui permettant d'hybrider une zone dite variable de ladite séquence prédéterminée, identifiée précédemment, cette zone variable étant spécifique à au moins une espèce du genre *Mycobacterium.*

Selon une caractéristique importante, la présente invention propose en outre des sondes spécifiques de groupes d'espèces correspondant aux complexes *M. tuberculosis* et *M. avium-intracellulare,* respectivement. Ces sondes comprennent une séquence nucléotidique leur permettant d'hybrider une zone variable du fragment dont la séquence est incluse dans le gène, codant pour 65 kD, ladite zone étant commune à plusieurs espèces d'un même complexe.

Préférentiellement, ces sondes d'espèce ou de complexe comprennent de 10 à 40 monomères.

Avantageusement, la séquence nucléotidique de la sonde est choisie parmi SEQ ID N012 et SEQ ID N013 à SEQ ID N021, identifiées en fin de description.

Selon l'invention, on propose un réactif ou ensemble de réactifs pour détecter sélectivement dans un échantillon biologique, une bactérie du genre Mycobacterium, appartenant aux complexes M. tuberculosis et M.avium-intracellulare. Un tel ensemble comprend, éventuellement une ou plusieurs amorces telles que décrites précédemment, éventuellement une ou plusieurs sondes de genre telles que décrites précédemment, et une ou plusieurs sondes d'espèces telles que décrites précédemment.

Selon la technique d'hybridation utilisée, la ou les sondes selon l'invention sont en milieu liquide et/ou fixées sur un support solide, directement ou indirectement. S'agissant du support solide selon l'invention, sous toutes formes appropriées telles que tube, cône, puits, plaque de microtitration, feuille, ou polymère soluble, il est choisi parmi les polystyrènes, les copolymères styrène-butadiène, les copolymères styrène-butadiène en mélange avec des polystyrènes, des polypropylènes, des polycarbonates, des copolymères polystyrène-acrylonitrile, des copolymères styrène-méthylméthacrylate de méthyle, parmi les fibres synthétiques et naturelles, parmi les polysaccharides et les dérivés de la cellulose.

L'invention concerne aussi un procédé pour détecter sélectivement dans un échantillon biologique une bactérie du genre mycobactérie. Ce procédé présente les étapes suivantes :
- préparation des échantillons afin de libérer l'acide nucléique mycobactérien
- hybridation de l'ADN et/ou d'ARN génomique de la bactérie, et/ou de son ARN de transcription, avec au moins une amorce telle que définie précédemment multiplication du fragment d'ADN ou ARN flanqué de ladite ou desdites amorces, pour obtenir une multitude de fragments monocaténaires d'ADN et/ou ARN, répondant à la définition selon l'invention ; ces deux étapes sont éventuelles, et ont pour but d'éviter la culture des bactéries

- exposition du fragment ou des fragments à au moins une sonde d'espèce, et/ou au moins une sonde de genre, telles que définies précédemment.

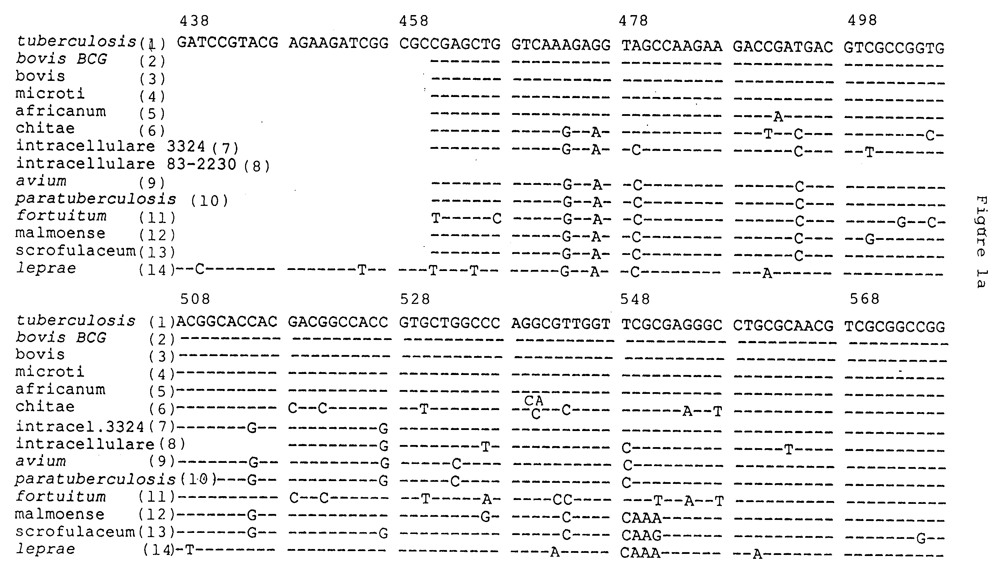

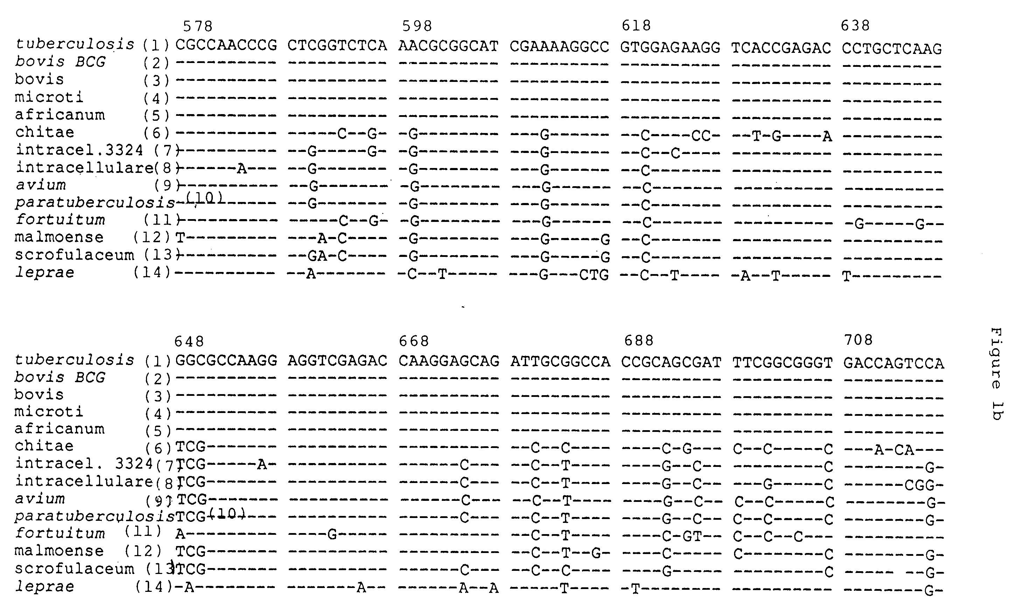

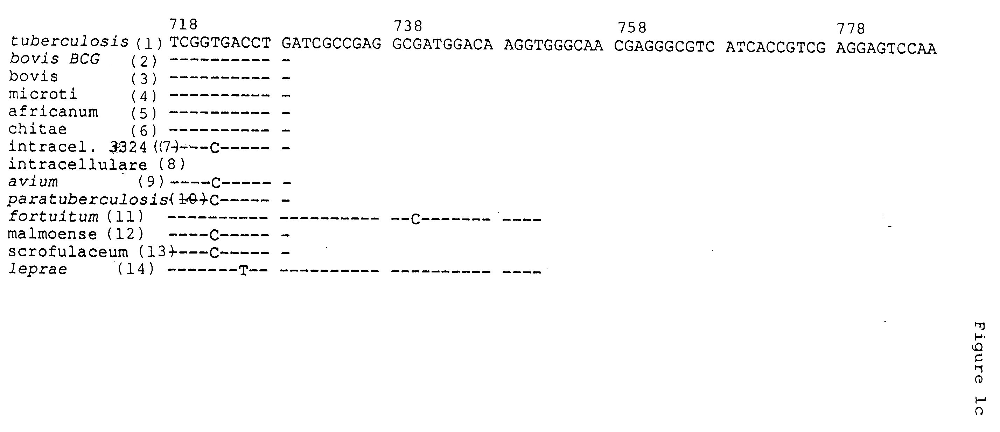

La présente invention est maintenant exposée selon les exemples 1 à 5, et à l'appui des tableaux 1 et 2 et de la figure 1, décomposée en figure 1a, 1b, et 1c, et qui représente l'alignement des séquences nucléotidiques (selon la numérotation de Shinnick et al. 1987) sur une portion de 314 bp du gène codant pour l'antigène 65 kD des mycobactéries ; les séquences des souches des espèces suivantes sont issues de la littérature :

*M. tuberculosis* (Document WO-A-8806591 et Shinnick, T.M. 1987. The 65-kilodalton antigen of *Mycobacterium tuberculosis.* J. Bacteriol. 169:1080-88)

*M. bovis BCG* (Thole et al. 1987. Characterization, sequence determination, and immunogenicity of a 64-kilodalton protein of *Mycobacterium bovis* BCG expressed in *Escherichia coli* K-12. Inf. Imm. 55:1466-1475).

*M. avium, M. fortuitum* et *M. paratuberculosis* (Document FR-A-2 645 878, et Hance, A.J., Grandchamp, B., Lévy-Frébault, V., Lecossier, D., Rauzier, J., Bocart, D., and Gicquel, B. 1989. Detection and identification of mycobacterium by amplification of mycobacterial DNA. Molecul. Microbiol. 3:843-9.)

*Mycobacterium leprae* (Young, R.A., Mehra, V., Sweeetser, D., Buchanan, T, Clark-Curtiss, J., Davis, R.W., and Bloom, B.R. 1985. Genes for the major protein antigens of the leprosy parasite *Mycobacterium leprae.* Nature. 316:450-2)

Les séquences des espèces suivantes ont été déterminées à partir de souches disponibles chez le déposant : *M. africanum, M. microti, M. chitae, M. intracellulare* 3324, *M. intracellulare* 83 2230, *M. malmoense, M. scrofulaceum.*

Selon les figures 1a à 1c un tiret signifie que la base est identique à celle identifiée en première ligne.

Le Tableau 1 représente des séquences oligonucléotidiques selon l'invention, exemplifiées ci-après, et leur spécificité.

Tableau 1

Oligonucléotides de synthèse utilisés en tant qu'amorces de polymérisation ou de sonde pour détecter une portion du gène de l'antigène 65 kD des mycobactéries, et dont la spécificité est de niveau genre ou espèces de mycobactéries

(*) les chiffres entre parenthèses indiquent la position de l'oligonucléotide selon la numérotation de Shinnick et al., 1987, C· indique une homologie avec le brin complémentaire.

| Oligonucléotide | Séquence* |
|---|---|

Amorces de polymérisation ou amplification
SEQ ID NO8: GAT CCG TAC GAG AAG ATC GG (438-457)
SEQ ID NO9: ACC TTG TCC ATC GCC TCG G  (C·733-751)

Sondes d'hybridation de genre :
SEQ ID NO11: CGC AAC GTC GCG GCC GGC GCC AAC CCG C (561-588)
SEQ ID NO10: CCG AGG CGA TGG ACA AGG T (733-751)

Sondes d'hybridation du complexe avium-intracellulare :
SEQ ID NO21 : TGC TCA AGT CGG CCA AGG (640-657)
SEQ ID NO13 : ACG GCA CGA CGA CGG (508-522)
SEQ ID NO14 : CCA CGG TGC TSG CYC AGG (523-540)
SEQ ID NO15 : GAC CAG YSG ATC GGC GAC C (708-726)
SEQ ID NO16 : CCG CTG GGT CTS AA (585-598)
SEQ ID NO17 : GCG TTG GTC CGC GAG GGC C (540-558)
SEQ ID NO18 : CGA CGA CGG CCA CGG TGC T (514-532)
SEQ ID NO19 : CCG CTG GGT CTS AAG CGC G (585-603)
SEQ ID NO20 : CCA AMC CGC TGG GTC TSA A (580-598)

Sonde d'hybridation du complexe tuberculosis :
SEQ ID NO12 : GGT CAA AGA GGT AGC CAA G (467-485)

Le Tableau 2 représente les spécificités d'amplification enzymatique et d'hybridation des souches bactériennes testées. Dans ce tableau :
- s'agissant de l'origine, les chiffres 1 réfèrent à des souches disponibles auprès du Centre de Collection des Mycobactéries, CHUV à Lausanne, 2 au Centre Médical Universitaire de Genève, 3 à l'Institut d'Hygiène de Genève, et Bio M aux laboratoires de bioMérieux
- TB1 et TB2 réfèrent à des amorces selon le document WO-A-90 12875.

EP 0 584 023 A1

Tableau 2

| ESPECE | ORIGINE | AMPLIFICATION | | HYBRIDATION DU PRODUIT D'AMPLIFICATION, et marquage de la sonde 32P radioactif (32P) ou détection enzymatique (froid) | | |
|---|---|---|---|---|---|---|
| | | TB 1 - TB 2 | SEQ ID No 8. SEQ ID No 9 | GENRE SEQ ID No 11 (32 P) | GENRE SEQ ID No 10 (froid) | Complexe M. tuberculosis SEQ ID No 12 (froid) |
| M. tuberculosis | 2 | + | + | + | + | + |
| M. microti | 3 | + | + | . + | + | + |
| M. bovis | 3 | + | + | + | + | + |
| M. africanum La 1077 | 1 | NE | + | + | + | + |
| M. kansasii | 3 | + | + | + | + | NE |
| M. marinum  2417 | 3 | + | + | . + | + | NE |
| M. simiae  ATCC 25275 | BioM | NE | + | + | + | . |
| M. scrofulaceum | 3 | + | + | + | + | NE |
| M. gordonae | BioM | + | + | + | + | . |
| M. szulgai | 3 | + | + | + | + | NE |
| M. flavescens | BioM | NE | + | + | + | . |
| M. intracellulare  83-2230 | 2 | . | + | + | + | NE |
| M. intracellulare  83-3324 | 2 | . | + | + | + | . |
| M. intracellulare ATCC 357 64 | BioM | NF | + | + | + | . |
| M. avium | 2 | + | + | + | + | NE |
| M. avium-intracellulare  4556 | 3 | + | + | + | + | . |
| M. xenopi  4333 | 3 | NF | + | + | + | NE. |
| M. terrae  ATCC 15755 | BioM | + | + | + | + | NE |
| M. malmoense | 3 | NE | + | + | + | NE |
| M. nonchromogenicum | 3 | NE | + | + | + | . |
| M. triviale  ATCC 23 292 | BioM | NE | + | + | + | NE |
| M. gastri | 1 | NE | + | NE | + | . |
| M. farcinogenes | 3 | NE | + | NE | + | NE |
| M. haemophilum | 1 | NE | + | NE | + | . |

**Tableau 2**

| | | | | | |
|---|---|---|---|---|---|
| M. ulcerans | 1 | + | | + | NE |
| M. paratuberculosis | 1 | + | | + | NE |
| M. chelonei | 2 BioM | NE | + | + | · |
| M. chelonei ATCC 14472 | 2 | + | + | + | NE |
| M. fortuitum 83-3359 | 2 | + | + | + | + |
| M. diernhoferi | 3 | + | + | + | + |
| M. chitae | 3 | + | + | + | + |
| M. chelonei-abscessus | 3 | + | + | + | NE |
| M. senegalese | 3 | + | + | + | NE |
| M. vaccae | 3 | + | + | + | · |
| M. phlei | 3 | + | + | + | + |
| M. thermoresistibile | 3 | + | + | + | · |
| M. smegmatis | 3 | + | + | + | · |
| Nocardia asteroides 864 | 2 | · | · | · | NE |
| Nocardia asteroides 866 | 2 | · | · | · | NE |
| Nocardia asteroides 869 | 2 | · | · | · | NE |
| Nocardia asteroides 870 | 2 | · | · | · | NE |
| Nocardia caviae 861 | 2 | · | · | · | NE |

1 centre de collection des mycobactéries, Lausanne, 2 Centre Médical Universitaire de Genève,
3 Institut d'hygiène de Genève

NE : non-effectué

**Exemple N°1 :**

Définition d'amorces d'amplification spécifiques de genre et leur utilisation

Après différentes expériences préliminaires, il a été constaté que les séquences proposées par la littérature, en tant qu'amorces "universelles" au genre Mycobacterium, ne répondent pas à certaines souches ou espèces connues à ce jour ; ainsi les amorces dites TB1 et TB2 selon le document WO-A-90 12875 ne répondent-elles pas à certaines espèces, comme indiqué précédemment.

Il a donc été procédé à un séquençage du gène codant pour l'antigène de 65 kD chez différentes espèces, dont ledit gène n'avait pas encore été exploré. Par alignement des séquences ainsi obtenues avec celles déjà décrites dans l'art antérieur, on a ensuite choisi des amorces nouvelles, dont on a découvert qu'elles hybridaient des séquences conservées chez pratiquement toutes les mycobactéries ; cf SEQ ID N08 et SEQ ID N09 du tableau 1. Cette découverte a été effectuée par amplification à partir d'une souche de mycobactéries. La technique utilisée est décrite ci-après.

L'extraction de l'ADN génomique a été conduite selon le protocole de Sjobring (Sjobring et al. 1990. Polymerase chain reaction for detection of *Mycobacterium tuberculosis*. J. Clin. Microbiol. 28(10):2200-2204). 200 ml de culture liquide sont centrifugés à 2600 t/mn pendant 15 mn. Le surnageant est écarté et les culots sont regroupés. On lave le culot avec du tampon pH 8.0 (50 mM Tris-base, 50 mM NaCl, 5mM EDTA pH 8.0). On ajoute 0,1 volume de tampon digestion 10x (100 mM Tris pH 8.0, 200 mM EDTA, SDS 10%) et la protéinase K à 5 mg/ml final. On incube à 60°C pendant 3 heures avec agitation et on chauffe 5 minutes à 100°C pour inactiver la protéinase K. L'ADN est précipité une première fois par ajout d'une solution de 0,4 volume de solution de bromure de cétyltrimethyl-ammonium (5% dans 0,4 M NaCl), on incube 15 minutes à température puis 15 mn à 4°C. On transfère dans des tubes Eppendorf et on centrifuge 15 mn à 12000 tpm. On écarte le surnageant et on lave le culot dans du tampon TE (Tris EDTA) (10 mM Tris pH 7,5, 1 mM EDTA). L'ADN est précipité une deuxième fois après une extraction au phénol-chloroforme (1:1)-alcool isoamylique (24:1). L'ADN est précipité avec 2 volumes d'éthanol absolu et 0,1 volume d'acétate de sodium 3 M.

L'ADN est amplifié selon la technique PCR de Saiki et al. (Saiki et al. 1988. Primer-directed enzymatic amplification of DNA with a thermostable DNA polymerase) à l'aide d'un appareil PCR Dri-Bock PCH-1 (TECHNE, Grande-Bretagne). Le milieu de réaction est constitué de Tris.Cl, 10 mmol/l; MgCl$_2$ 1,5 mmol/l; KCl 50 mmol/l; gélatine 1 mg/ml; dATP, dCTP, dGTP, dTTP 0,5 mmol/l chacun; pH 8,3; oligonucléotide TB1 (selon le document WO-A-90 12875, 25 pmol; oligonucléotide TB2 (selon le document WO-A-90 12875), 25 pmol et 10 µl de la préparation de l'ADN bactérien. Après dénaturation 5 mn suivie d'une centrifugation, on ajoute l'enzyme à 1,5 U/réaction. La PCR est effectuée sur 27 cycles avec les paramètres 96°C/43°C/74°C, pendant 1 mn, 1 mn, et 0,7 mn respectivement.

L'ADN amplifié est analysé par electrophorèse en gel d'agarose 0,8% en tampon TBE (Tris Borate EDTA) (89 mM Tris base, 89 mM acide borique, 2 mM EDTA). Les bandes sont visualisées par le bromure d'éthidium.

**Exemple N°2 :**

Détermination de séquences nucléotidiques de diverses espèces de mycobactéries et alignement de séquence.

A partir de l'ADN total isolé comme précédemment, une portion du gène codant pour l'antigène de 65 kD a été amplifiée à l'aide des amorces SEQ ID N08 et SZQ ID N09 du tableau 1. Les produits d'amplification obtenus ont été directement séquencés à l'aide du kit Gibco BRL (cyclage thermique). Les diverses séquences obtenues sont présentées dans la figure 1.

**Exemple N°3 :**

Détermination des sondes oligonucléotidiques spécifiques de genre et d'espèces et leur utilisation sur les échantillons d'ADN amplifiés selon l'exemple N° 1.

Les produits d'amplification par PCR obtenus à partir des amorces SEQ ID N08 et SEQ ID N09 (tableau 2) ont été testés avec diverses sondes d'espèces ou de genre décrites dans le tableau 1, selon la technique du Dot-Blot.

L'ADN substrat de la réaction d'amplification est extrait par une technique différente de celle de l'exemple 1: après centrifugation d'un aliquote de 10$^7$ mycobactéries en Tween 0,5%, les bactéries sont suspendues dans un tampon Tris. HCl (pH 8.0, 50 mM) puis sont soumises à sonication 10 mn à 55°C en présence de billes de verre 10 µm siliconées, puis à ébullition 5 mn. 10 µl du lysat sont ensuite amplifiés selon le protocole PCR décrit dans l'exemple n°1.

L'hybridation des oligonucléotides d'espèces ou de genre a été conduite selon le protocole décrit par Ausubel FM, Brent R, Kingston RE, Moore DD, Smith JA, Seidman JG et Struhl K (1987) Current protocols in molecular biology, Green publishing Associated and Wiley intersciences, New-York. Les oligonucléotides sont marqués par kination au [gamma(ou )-$^{32}$P] ATP (5000 Ci/mmol) et les températures et conditions de lavage sont les suivantes, par exemple pour MYC3 : 59°C, 30 mn en 1xSSC (Saline Sodium Citrate : 0,15 M NaCl, 0,015 M Na$_3$ citrate (2 H$_2$O pH 7.0) 1% SDS.

**Exemple N°4 :**

Typage d'espèces de mycobactéries par hybridation d'un produit d'amplification de genre mycobactérien à l'aide de sondes oligonucléotidiques, fait en utilisant un système de détection non-radioactif et semi-automatisé décrit dans le document FR-A-2 663 040.

Les produits d'amplification obtenus dans l'exemple n°3 ont été à nouveau testés selon la technologie sonde froide décrite ci-aprés selon deux variantes.

La première technique utilise un format plaque de microtitration

Dans une plaque de microtitration (Nunc 439454) est déposée une solution de l'oligonucléotide de capture à 1 ng/µl dans du PBS 3X (0.45 M NaCl 0.15 M phosphate de sodium pH 7.0). L'oligonucléotide ou sonde de capture est choisi parmi les oligonucléotides décrits dans le tableau 2 (SEQ ID N011, SEQ ID N010, SEQ ID N012, SEQ ID N021 et SEQ ID N013 à SEQ ID N020), spécifiques du genre ou de diverses espèces de mycobactéries. Leur position dans la séquence est indiquée dans la Figure 1. La plaque est incubée 2 h à 37° C, puis lavée 3 fois avec 300 μl de PBST (PBS 1x, Tween 20 0,5% (Merck 822184)). La cible constituée par 4 μl du produit amplifié est mélangée avec 76 μl de tampon PBS saumon (PBS3X + ADN de sperme de saumon 10 μg/ml (Sigma D9156) et 10 μl de soude 2N. L'ensemble est neutralisé 5 mn aprés par l'addition de 10 μl d'acide acétique 2N. L'ensemble est ajouté dans le puits en plus de 50 μl d'une solution du conjugué oligonucléotide-péroxydase à la concentration de 0.5 ng/μl en oligonucléotide dans un tampon PBS cheval (PBS3X + 10% sérum de cheval (BioMérieux 55842). Le conjugué oligonucléotide-péroxydase constitue la sonde de détection et possède la séquence nucléotidique de SES ID N010. La plaque est incubée 1 h à 37° C et lavée par 3 X 300 μl de PBST. Dans chaque puits, on rajoute 100 μl de substrat OPD (ortho-phenylenediamine Cambridge Medical Biotechnology ref/456 ) dans un tampon adapté (0,055 M acide citrique, 0.1 M $Na_2HPO_4$, pH 4,93) à la concentration de 4 mg/ml, auquel on ajoute extemporanément $H_2O_2$ à 30 volumes au 1/1000. Après 20 mn de réaction l'activité enzymatique est bloquée par 100 μl d'$H_2SO_4$ 1N et la lecture est effectuée sur un appareil Axia Microreader (Axia, marque déposée par BioMérieux) à 492 nm.

Les résultats de spécificité sont mentionnés dans le tableau 2. Ils indiquent que la sonde MYC 2-S est spécifique du genre mycobactéries, que la sonde TUB 1-S est spécifique uniquement des espèces du complexe *tuberculosis.* Par ailleurs la sonde peut être marquée à l'aide d'un isotope radioactif, d'une enzyme appropriée, d'un fluorochrome, d'un analogue de base, ou d'un composé impliqué dans une réaction de luminescence. En outre la sonde peut avoir un squelette ester de diphosphate, alkyl ou acrylphosphonate ou phosphorothioate ou de nature polyamide.

La seconde technique utilise le format de l'automate VIDAS (marque déposée par bioMérieux, France).

(1) INFORMATIONS GENERALES:

(i) DEPOSANT: BIO MERIEUX

(ii) TITRE DE L'INVENTION: Fragment d'ADN de mycobactéries, amorces d'amplification, sondes d'hybridation, réactifs et procédé de détection de mycobactéries

(iii) NOMBRE DE SEQUENCES: 21

(iv) ADRESSE DE CORRESPONDANCE:
(A) NOM: Cabinet GERMAIN & MAUREAU
(B) RUE: 20 Bd Eugène Deruelle
(C) VILLE: LYON
(D) PAYS: FRANCE
(E) CODE POSTAL: 69003
(F) TELEPHONE: 78 60 24 93
(G) TELEFAX: 78 60 92 85

(v) FORME DECHIFFRABLE PAR ORDINATEUR:
(A) TYPE DE SUPPORT: disquette 3,5 pouces DS, HD
(B) ORDINATEUR: EPSON (compatible IBM)
(C) SYSTEME D'EXPLOITATION: DOS
(D) LOGICIEL: MICROSOFT WORD POUR WINDOWS

(vi) DEMANDE ACTUELLE:
(A) DATE DE LA DEMANDE: 12.08.1993
(B) NUMERO DE LA DEMANDE:

(vii) DATE DE LA DEMANDE ANTERIEURE: 12.08.92
(A) NUMERO DE LA DEMANDE: 92 10094
(B) CLASSIFICATION: C07K, C12Q, C12N, A61K

(viii) MANDATAIRE:
(A) NOM: CABINET GERMAIN & MAUREAU, Philippe MAUREAU
(B) REFERENCE DOSSIER: MD/B05B1550EP

2) INFORMATIONS CONCERNANT SEQ ID NO:1:

i) CARACTERISTIQUES DE LA SEQUENCE:

A) LONGUEUR: 268 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME: Mycobacterium bovis

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 461-728

C) METHODE D'IDENTIFICATION:

D) AUTRES RENSEIGNEMENTS:

xi) DESCRIPTION DE LA SEQUENCE:

SEQ ID NO 1:

```
CGAGCTGGTC AAAGAGGTAG CCAAGAAGAC CGATGACGTC GCCGGTGACG GCACCACGAC   60
GGCCACCGTG CTGGCCCAGG CGTTGGTTCG CGAGGGCCTG CGCAACGTCG CGGCCGGCGC  120
CAACCCGCTC GGTCTCAAAC GCGGCATCGA AAAGGCCGTG GAGAAGGTCA CCGAGACCCT  180
GCTCAAGGGC GCCAAGGAGG TCGAGACCAA GGAGCAGATT GCGGCCACCG CAGCGATTTC  240
GGCGGGTGAC CAGTCCATCG GTGACCTG                                     268
```

2) INFORMATIONS CONCERNANT SEQ ID NO:2:

i) CARACTERISTIQUES DE LA SEQUENCE:

A) LONGUEUR: 268 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME: Mycobacterium microti

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 461-728

C) METHODE D'IDENTIFICATION:

D) AUTRES RENSEIGNEMENTS:

xi) DESCRIPTION DE LA SEQUENCE:

SEQ ID NO 2 :

```
CGAGCTGGTC AAAGAGGTAG CCAAGAAGAC CGATGACGTC GCCGGTGACG GCACCACGAC   60
GGCCACCGTG CTGGCCCAGG CGTTGGTTCG CGAGGGCCTG CGCAACGTCG CGGCCGGCGC  120
CAACCCGCTC GGTCTCAAAC GCGGCATCGA AAAGGCCGTG GAGAAGGTCA CCGAGACCCT  180
GCTCAAGGGC GCCAAGGAGG TCGAGACCAA GGAGCAGATT GCGGCCACCG CAGCGATTTC  240
GGCGGGTGAC CAGTCCATCG GTGACCTG                                     268
```

2) INFORMATIONS CONCERNANT SEQ ID NO:3:

i) CARACTERISTIQUES DE LA SEQUENCE:
A) LONGUEUR: 268 paires de bases
B) TYPE: acide nucléique
C) NOMBRE DE BRINS:
D) CONFIGURATION:

ii) TYPE DE MOLECULE: ADN (génomique)

iii) HYPOTHETIQUE:

iv) ANTI-SENS:

vi) ORIGINE:
A) ORGANISME: Mycobacterium africanum
B) SOUCHE:
C) INDIVIDU/ISOLE:

viii) POSITION DANS LE GENOME:
A) CHROMOSOME/SEGMENT:
B) POSITION SUR LA CARTE:

ix) CARACTERISTIQUE:
A) NOM/CLE:
B) EMPLACEMENT: 461-728
C) METHODE D'IDENTIFICATION:
D) AUTRES RENSEIGNEMENTS:

xi) DESCRIPTION DE LA SEQUENCE:
SEQ ID NO 3:

```
CGAGCTGGTC AAAGAGGTAG CCAAGAAGAC CAATGACGTC GCCGGTGACG GCACCACGAC   60
GGCCACCGTG CTGGCCCAGG CGTTGGTTCG CGAGGGCCTG CGCAACGTCG CGGCCGGCGC  120
CAACCCGCTC GGTCTCAAAC GCGGCATCGA AAAGGCCGTG GAGAAGGTCA CCGAGACCCT  180
GCTCAAGGGC GCCAAGGAGG TCGAGACCAA GGAGCAGATT GCGGCCACCG CAGCGATTTC  240
GGCGGGTGAC CAGTCCATCG GTGACCTG                                     268
```

2) INFORMATIONS CONCERNANT SEQ ID NO:4:


i) CARACTERISTIQUES DE LA SEQUENCE:

A) LONGUEUR: 268 paires de bases

B) TYPE: acide nucléique

C) NOMBRE DE BRINS:

D) CONFIGURATION:


ii) TYPE DE MOLECULE: ADN (génomique)


iii) HYPOTHETIQUE:


iv) ANTI-SENS:


vi) ORIGINE:

A) ORGANISME: Mycobacterium chitae

B) SOUCHE:

C) INDIVIDU/ISOLE:


viii) POSITION DANS LE GENOME:

A) CHROMOSOME/SEGMENT:

B) POSITION SUR LA CARTE:


ix) CARACTERISTIQUE:

A) NOM/CLE:

B) EMPLACEMENT: 461-728

C) METHODE D'IDENTIFICATION:

D) AUTRES RENSEIGNEMENTS:


xi) DESCRIPTION DE LA SEQUENCE:

SEQ ID NO 4:

```
CGAGCTGGTC AAGGAAGTAG CCAAGAAGAC TGACGACGTC GCCGGCGACG GCACCACCAC   60
CGCCACCGTT CTGGCCCASV CGCTGGTTCG CGAAGGTCTG CGCAACGTCG CGGCCGGCGC  120
CAACCCGCTC GGCCTGAAGC GCGGCATCGA GAAGGCCGTC GAGACCGTCT CGGAGAACCT  180
GCTCAAGTCG GCCAAGGAGG TCGAGACCAA GGAGCAGATC GCCGCCACCG CCGGGATCTC  240
CGCGGGCGAC AACACCATCG GTGACCTG                                    268
```

2) INFORMATIONS CONCERNANT SEQ ID NO:5:

i) CARACTERISTIQUES DE LA SEQUENCE:

A) LONGUEUR: 268 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME: Mycobacterium intracellulare

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 461-728

C) METHODE D'IDENTIFICATION:

D) AUTRES RENSEIGNEMENTS:

xi) DESCRIPTION DE LA SEQUENCE:

SEQ ID NO 5:

```
CGAGCTGGTC AAGGAAGTCG CCAAGAAGAC CGACGACGTT GCCGGTGACG GCACGACGAC   60
GGCCACGGTG CTGGCCCAGG CGTTGGTTCG CGAGGGCCTG CGCAACGTCG CGGCCGGCGC  120
CAACCCGCTG GGTCTGAAGC GCGGCATCGA GAAGGCCGTC GACAAGGTCA CCGAGACCCT  180
GCTCAAGTCG GCCAAAGAGG TCGAGACCAA GGACCAGATC GCTGCCACCG CGGCCATTTC  240
GGCGGGCGAC CAGTCGATCG GCGACCTG                                      268
```

2) INFORMATIONS CONCERNANT SEQ ID NO:6:

i) CARACTERISTIQUES DE LA SEQUENCE:

A) LONGUEUR: 200 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME: Mycobacterium intracellulare

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 518-717

C) METHODE D'IDENTIFICATION:

D) AUTRES RENSEIGNEMENTS:

xi) DESCRIPTION DE LA SEQUENCE:

SEQ ID NO 6:

```
GACGGCCACG GTGCTGGCTC AGGCGTTGGT CCGCGAGGGC CTGCGTAACG TCGCGGCCGG   60
CGCCAAACCG CTGGGTCTCA AGCGCGGCAT CGAGAAGGCC GTCGAGAAGG TCACCGAGAC  120
CCTGCTCAAG TCGGCCAAGG AGGTCGAGAC CAAGGACCAG ATCGCTGCCA CCGCGGCCAT  180
TTCGGCGGGC GACCAGCGGA                                             200
```

2) INFORMATIONS CONCERNANT SEQ ID NO:7:

i) CARACTERISTIQUES DE LA SEQUENCE:
A) LONGUEUR: 268 paires de bases
B) TYPE: acide nucléique
C) NOMBRE DE BRINS:
D) CONFIGURATION:

ii) TYPE DE MOLECULE: ADN (génomique)

iii) HYPOTHETIQUE:

iv) ANTI-SENS:

vi) ORIGINE:
A) ORGANISME:
B) SOUCHE:
C) INDIVIDU/ISOLE:

viii) POSITION DANS LE GENOME:
A) CHROMOSOME/SEGMENT:
B) POSITION SUR LA CARTE:

ix) CARACTERISTIQUE:
A) NOM/CLE:
B) EMPLACEMENT: 461-728
C) METHODE D'IDENTIFICATION:
D) AUTRES RENSEIGNEMENTS:

xi) DESCRIPTION DE LA SEQUENCE:
SEQ ID NO 7:

```
CGAGCTGGTC AAGGAAGTCG CCAAGAAGAC CGACGACGTC GCCGGTGACG GCACGACGAC   60
GGCCACGGTG CTGGCCCAGG CGCTGGTCAA GGAGGGCCTG CGCAACGTCG CGGCGGGCGC  120
CAACCCGCTG AGCCTCAAGC GCGGCATCGA GAAGGCGGTC GAGAAGGTCA CCGAGACCCT  180
GCTCAAGTCG GCCAAGGAGG TCGAGACCAA GGACCAGATC GCCGCCACCG CGGCGATTTC  240
GGCGGGCGAC CAGTCGATCG GCGACCTG                                     268
```

2) INFORMATIONS CONCERNANT SEQ ID NO:8:

i) CARACTERISTIQUES DE LA SEQUENCE:

A) LONGUEUR: 20 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME:

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 438-457

C) METHODE D'IDENTIFICATION:

D) AUTRES RENSEIGNEMENTS:

xi) DESCRIPTION DE LA SEQUENCE:

SEQ ID NO 8:

GATCCGTACG AGAAGATCGG                                    20

2) INFORMATIONS CONCERNANT SEQ ID NO:9:

i) CARACTERISTIQUES DE LA SEQUENCE:

A) LONGUEUR: 19 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME:

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 733-751

C) METHODE D'IDENTIFICATION:

D) AUTRES RENSEIGNEMENTS:

xi) DESCRIPTION DE LA SEQUENCE:

SEQ ID NO 9:
ACCTTGTCCA TCGCCTCGG                                                              19

2) INFORMATIONS CONCERNANT SEQ ID NO:10:

i) CARACTERISTIQUES DE LA SEQUENCE:
A) LONGUEUR: 19 paires de bases
B) TYPE: acide nucléique
C) NOMBRE DE BRINS:
D) CONFIGURATION:

ii) TYPE DE MOLECULE: ADN (génomique)

iii) HYPOTHETIQUE:

iv) ANTI-SENS:

vi) ORIGINE:
A) ORGANISME:
B) SOUCHE:
C) INDIVIDU/ISOLE:

viii) POSITION DANS LE GENOME:
A) CHROMOSOME/SEGMENT:
B) POSITION SUR LA CARTE:

ix) CARACTERISTIQUE:
A) NOM/CLE:
B) EMPLACEMENT: 733-751
C) METHODE D'IDENTIFICATION:
D) AUTRES RENSEIGNEMENTS:

xi) DESCRIPTION DE LA SEQUENCE:
SEQ ID NO 10:
CCGAGGCGAT GGACAAGGT                                    19

2) INFORMATIONS CONCERNANT SEQ ID NO:11:

i) CARACTERISTIQUES DE LA SEQUENCE:
A) LONGUEUR: 28 paires de bases
B) TYPE: acide nucléique
C) NOMBRE DE BRINS:
D) CONFIGURATION:

ii) TYPE DE MOLECULE: ADN (génomique)

iii) HYPOTHETIQUE:

iv) ANTI-SENS:

vi) ORIGINE:
A) ORGANISME:
B) SOUCHE:
C) INDIVIDU/ISOLE:

viii) POSITION DANS LE GENOME:
A) CHROMOSOME/SEGMENT:
B) POSITION SUR LA CARTE:

ix) CARACTERISTIQUE:
A) NOM/CLE:
B) EMPLACEMENT: 561-588
C) METHODE D'IDENTIFICATION:
D) AUTRES RENSEIGNEMENTS:

xi) DESCRIPTION DE LA SEQUENCE:
SEQ ID NO 11:
CGCAACGTCG CGGCCGGCGC CAACCCGC                                                28

2) INFORMATIONS CONCERNANT SEQ ID NO:12:

i) CARACTERISTIQUES DE LA SEQUENCE:

A) LONGUEUR: 19 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME:

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 467-485

C) METHODE D'IDENTIFICATION:

D) AUTRES RENSEIGNEMENTS:

xi) DESCRIPTION DE LA SEQUENCE:

SEQ ID NO 12:

GGTCAAAGAG GTAGCCAAG                                                    19

2) INFORMATIONS CONCERNANT SEQ ID NO:13:

i) CARACTERISTIQUES DE LA SEQUENCE:

A) LONGUEUR: 15 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME:

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 508-522

C) METHODE D'IDENTIFICATION:

D) AUTRES RENSEIGNEMENTS:

xi) DESCRIPTION DE LA SEQUENCE:

SEQ ID NO 13:

ACGGCACGAC GACGG                                                              15

2) INFORMATIONS CONCERNANT SEQ ID NO:14:

i) CARACTERISTIQUES DE LA SEQUENCE:

A) LONGUEUR: 18 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME:

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 523-540

C) METHODE D'IDENTIFICATION:

D) AUTRES RENSEIGNEMENTS:

xi) DESCRIPTION DE LA SEQUENCE:

SEQ ID NO 14:

CCACGGTGCT SGCYCAGG                                                   18

2) INFORMATIONS CONCERNANT SEQ ID NO:15:

i) CARACTERISTIQUES DE LA SEQUENCE:

A) LONGUEUR: 19 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME:

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 708-726

C) METHODE D'IDENTIFICATION:

D) AUTRES RENSEIGNEMENTS:

xi) DESCRIPTION DE LA SEQUENCE:

SEQ ID NO 15:

GACCAGYSGA TCGGCGACC                                                    19

2) INFORMATIONS CONCERNANT SEQ ID NO:16:

i) CARACTERISTIQUES DE LA SEQUENCE:

A) LONGUEUR: 14 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME:

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 585-598

C) METHODE D'IDENTIFICATION:

D) AUTRES RENSEIGNEMENTS:

xi) DESCRIPTION DE LA SEQUENCE:

SEQ ID NO 16:

CCGCTGGGTC TSAA                                                          14

2) INFORMATIONS CONCERNANT SEQ ID NO:17:

i) CARACTERISTIQUES DE LA SEQUENCE:

A) LONGUEUR: 19 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME:

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 540-558

C) METHODE D'IDENTIFICATION:

D) AUTRES RENSEIGNEMENTS:

xi) DESCRIPTION DE LA SEQUENCE:

SEQ ID NO 17:

GCGTTGGTCC GCGAGGGCC                                                        19

2) INFORMATIONS CONCERNANT SEQ ID NO:18:

i) CARACTERISTIQUES DE LA SEQUENCE:

A) LONGUEUR: 19 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME:

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 514-532

C) METHODE D'IDENTIFICATION:

D) AUTRES RENSEIGNEMENTS:

xi) DESCRIPTION DE LA SEQUENCE:

SEQ ID NO 18:

CGACGACGGC CACGGTGCT                                                                 19

2) INFORMATIONS CONCERNANT SEQ ID NO:19:

i) CARACTERISTIQUES DE LA SEQUENCE:

A) LONGUEUR: 19 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME:

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 585-603

C) METHODE D'IDENTIFICATION:

D) AUTRES RENSEIGNEMENTS:

xi) DESCRIPTION DE LA SEQUENCE:

SEQ ID NO 19:

CCGCTGGGTC TSAAGCGCG                                              19

2) INFORMATIONS CONCERNANT SEQ ID NO:20:

i) CARACTERISTIQUES DE LA SEQUENCE:

A) LONGUEUR: 19 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME:

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 580-598

C) METHODE D'IDENTIFICATION:

D) AUTRES RENSEIGNEMENTS:

xi) DESCRIPTION DE LA SEQUENCE:

SEQ ID NO 20:

CCAAMCCGCT GGGTCTSAA                                                      19

2) INFORMATIONS CONCERNANT SEQ ID NO:21:

i) CARACTERISTIQUES DE LA SEQUENCE:

A) LONGUEUR: 18 paires de bases

B) TYPE: acide nucléique

C) NOMBRE DE BRINS:

D) CONFIGURATION:

ii) TYPE DE MOLECULE: ADN (génomique)

iii) HYPOTHETIQUE:

iv) ANTI-SENS:

vi) ORIGINE:

A) ORGANISME:

B) SOUCHE:

C) INDIVIDU/ISOLE:

viii) POSITION DANS LE GENOME:

A) CHROMOSOME/SEGMENT:

B) POSITION SUR LA CARTE:

ix) CARACTERISTIQUE:

A) NOM/CLE:

B) EMPLACEMENT: 640-657

C) METHODE D'IDENTIFICATION:

D) AUTRES RENSEIGNEMENTS:

xi) DESCRIPTION DE LA SEQUENCE:

SEQ ID NO 21:

TGCTCAAGTC GGCCAAGG                                                    18

## Revendications

1/ Fragment nucléotidique d'ADN, dont la séquence nucléotidique est incluse dans le gène des espèces du genre *Mycobacterium,* codant pour l'antigène mycobactérien 65 kD, comportant des régions homologues à pratiquement toutes les espèces du genre Mycobacterium, et au moins une région variable spécifique à l'espèce, **caractérisé en ce que** ledit fragment est choisi parmi les fragments dont les séquences nucléotidiques présentent au moins 70 % d'homologie, et préférentiellement au moins 85 % d'homologie, avec une séquence prédéterminée ou sa séquence complémentaire, ladite séquence prédéterminée commençant au nucléotide 438 et finissant au nucléotide 751 dudit gène codant pour ledit antigène de toutes les espèces de mycobactéries à l'exclusion des espèces *M. tuberculosis, M. bovis BCG, M. avium, M. paratuberculosis, M. fortuitum, M. malmoense, M. laprea, M. kansaii et M. marinum.*

2/ Fragment selon la revendication 1, caractérisé en ce qu'il est choisi parmi les fragments dont les séquences nucléotidiques présentent au moins 70 % d'homologie, et préférentiellement au moins 85 % d'homologie, avec l'une quelconque des séquences SEQ ID N01, SEQ ID N02, SEQ ID N03, SEQ ID N04, SEQ ID N05, SEQ ID N06 et SEQ ID N07.

3/ ADN génomique ou isolé, et/ou ARN, notamment vecteur de réplication, comprenant ou intégrant un fragment selon l'une quelconque des revendications 1 à 2.

4/ Amorce spécifique pour l'amplification par polymérisation de l'ADN génomique d'une bactérie du genre mycobactérie, caractérisée ce qu'elle comprend une séquence nucléotidique lui permettant d'hybrider une zone dite constante de la séquence prédéterminée définie à la revendication 1, homologue à pratiquement toutes les espèces du genre mycobactéries.

5/ Amorce selon la revendication 4, caractérisée en ce qu'elle comprend entre 15 et 25 bases.

6/ Amorce selon la revendication 4, caractérisée en ce qu'elle présente une séquence nucléotidique choisie parmi SEQ ID N08 et SEQ ID N09.

7/ Couple d'amorces, caractérisé en ce qu'il comprend deux amorces choisies parmi les amorces selon la revendication 4.

8/ Sonde de genre, caractérisée en ce qu'elle est susceptible d'hybrider une zone constante d'un fragment monocaténaire selon l'une quelconcque des revendications 1 à 3, homologue à pratiquement toutes les espèces du genre *Mycobacterium.*

9/ Sonde de genre, caractérisée en ce qu'elle est susceptible d'hybrider une zone extrême d'un fragment monocaténaire selon l'une quelconque des revendications 1 à 3, correspondant à une amorce selon la revendication 5, intégrée dans ledit fragment.

10/ Sonde selon la revendication 9, caractérisée en ce qu'elle présente la séquence nucléotidique SEQ ID N010 et SEQ ID N011.

11/ Sonde d'espèce, caractérisée en ce qu'elle comprend une séquence nucléotidique lui permettant d'hybrider une zone dite variable de la séquence prédéterminée définie à la revendication 1, spécifique d'au moins une espèce du genre *Mycobacteriuim.*

12/ Sonde selon l'une quelconque dess revendications 8 à 11, caractérisée en ce qu'elle comprend 10 à 40 monomères.

13/ Sonde spécifique du groupe des espèces du complexe *M. tuberculosis* comprenant une séquence nucléotidique, susceptible d'hybrider une zone du fragment dont la séquence nucléotidique est incluse dans le gène des espèces du complexe *M. tuberculosis,* codant pour l'antigène 65 kD, caractérisée en ce qu'elle possède une séquence nucléotidique présentant au moins 70 % d'homologie et de préférence au moins 85 % d'homologie avec la séquence SEQ ID N012 ou sa séquence complémentaire.

14/ Sonde spécifique du groupe des espèces du complexe *M. avium-intracellulare* (MAIC) comprenant une séquence nucléotidique susceptible d'hybrider une zone du fragment dont la séquence nucléotidique est incluse dans le gène des espèces du complexe MAIC, codant pour l'antigène 65 kD, caractérisée en ce qu'elle possède une séquence nucléotidique présentant au moins 70 % d'homologie et préférentiellement au moins 85 % d'homologie avec les séquences choisies parmi SEQ ID N013, SEQ ID N014, SEQ ID N015, SEQ ID N016, SEQ ID N017, SEQ ID N018, SEQ ID N019, SEQ ID N020, SEQ ID N021.

15/ Ensemble de réactifs pour détecter sélectivement dans un échantillon biologique une bactérie du genre *Mycobacterium,* appartenant notamment aux complexes *M. tuberculosis* et *M avium-intracellulare,* caractérisé en ce qu'il comprend, d'une part au moins une amorce selon l'une quelconque des revendications 4 à 6, et d'autre part au moins une sonde d'espèces selon la revendication 13 ou 14, et éventuellement une sonde de genre selon la revendication 9 ou 10.

16/ Ensemble selon la revendication 15, caractérisé en ce que la ou les sondes sont en milieu liquide et/ou fixées sur un support solide directement ou indirectement.

17/ Ensemble selon la revendication 16, caractérisé en ce qu'il associe une sonde de genre et une sonde d'espèce, et l'une des sondes est fixée sur un support solide, en tant que sonde de capture, tandis que l'autre sonde est en milieu liquide, en tant que sonde de détection.

18/ Ensemble selon la revendication 16, caractérisé en ce que la ou les sondes sont fixées sur un support solide choisi parmi les copolymères styrène-butadiène en mélange avec des polystyrènes, des polypropylènes, des polycarbonates, des copolymères polystyrène-acrylonitrile, des copolymères styrène-méthylméthacrylate de méthyle, parmi les fibres synthétiques et naturelles, parmi les polysaccharides et les dérivés de la cellulose.

19/ Procédé pour détecter sélectivement dans un échantillon biologique une bactérie du genre mycobactérie, caractérisé en ce qu'on hybride l'ADN génomique de la bactérie contenue dans ledit échantillon, et/ou son ARN de transcription avec au moins une amorce selon l'une quelconque des revendications 4 à 6, on multiplie le fragment d'ADN et/ou d'ARN ainsi flanqué de ladite amorce pour obtenir une multitude de fragments monocaténaires d'ADN et/ou ARN, selon l'une quelconque des revendications 1 à 2, et on expose lesdits fragments à une sonde d'espèce selon l'une quelconque des revendications 11 à 14, et éventuellement une sonde de genre selon l'une quelconque des revendications 9 et 10.

EP 0 584 023 A1

Figure 1a

```
                        438              458              478              498
tuberculosis(1)  GATCCGTACG AGAAGATCGG CGCCGAGCTG GTCAAAGAGG TAGCCAAGAA GACCGATGAC GTCGCCGGTG
bovis BCG    (2)                        ---------- ---------- ---------- ---------- ----------
bovis        (3)                        ---------- ---------- ---------- ---------- ----------
microti      (4)                        ---------- ---------- ---------- ---------- ----------
africanum    (5)                        ---------- ---------- ---------- ----A----- ----------
chitae       (6)                        -----G--A- ---------- --T--C--- --------C-
intracellulare 3324(7)                  -----G--A- -C-------- -----C--- --T-------
intracellulare 83-2230(8)
avium        (9)                        -----G--A- -C-------- -----C--- ----------
paratuberculosis (10)                   -----G--A- -C-------- -----C--- ----------
fortuitum    (11)         T-----C       -----G--A- -C-------- -----C--- -----G--C-
malmoense    (12)                       -----G--A- -C-------- -----C--- --G-------
scrofulaceum(13)                        -----G--A- -C-------- -----C--- ----------
leprae       (14) --C------- --------T-- ---T---T-- -----G--A- -C-------- ---A------ ----------

                       508              528              548              568
tuberculosis (1)ACGGCACCAC GACGGCCACC GTGCTGGCCC AGGCGTTGGT TCGCGAGGGC CTGCGCAACG TCGCGGCCGG
bovis BCG    (2)---------- ---------- ---------- ---------- ---------- ---------- ----------
bovis        (3)---------- ---------- ---------- ---------- ---------- ---------- ----------
microti      (4)---------- ---------- ---------- ---------- ---------- ---------- ----------
africanum    (5)---------- ---------- ---------- ---------- ---------- ---------- ----------
                                                            CA
chitae       (6)---------- C--C------ --T------- --C--C---- ------A--T ---------- ----------
intracel.3324(7)-------G-- ---------G ---------- ---------- ---------- ---------- ----------
intracellulare(8)         ---------G -------T- ---------- C--------- -----T---- ----------
avium        (9)-------G-- ---------G -----C---- ---------- C--------- ---------- ----------
paratuberculosis(10)---G-- ---------G -----C---- ---------- C--------- ---------- ----------
fortuitum    (11)---------- C--C------ --T-----A- ----CC---- ---T--A--T ---------- ----------
malmoense    (12)-------G-- ---------- -------G- -----C---- CAAA------ ---------- ----------
scrofulaceum(13)-------G-- ---------G ---------- -----C---- CAAG------ ---------- --------G--
leprae       (14)-T-------- ---------- ---------- ----A----- CAAA------ --A------- ----------
```

EP 0 584 023 A1

```
                       578                598                618                638
tuberculosis   (1) CGCCAACCCG CTCGGTCTCA AACGCGGCAT CGAAAAGGCC GTGGAGAAGG TCACCGAGAC CCTGCTCAAG
bovis BCG      (2) ---------- ---------- ---------- ---------- ---------- ---------- ----------
bovis          (3) ---------- ---------- ---------- ---------- ---------- ---------- ----------
microti        (4) ---------- ---------- ---------- ---------- ---------- ---------- ----------
africanum      (5) ---------- ---------- ---------- ---------- ---------- ---------- ----------
chitae         (6) ---------- -----C--G- -G-------- ---G------ --C----CC- --T-G----A ----------
intracel.3324  (7) ---------- --G----G- -G-------- ---G------ --C--C---- ---------- ----------
intracellulare (8) -------A--- --G------- -G-------- ---G------ --C------- ---------- ----------
avium          (9) ---------- --G------- -G-------- ---G------ --C------- ---------- ----------
paratuberculosis (10) ------- --G------- -G-------- ---G------ --C------- ---------- ----------
fortuitum      (11) ---------- -----C--G- -G-------- ---G------ --C------- ---------- -G-----G--
malmoense      (12) T--------- ---A-C---- -G-------- ---G----G --C------- ---------- ----------
scrofulaceum   (13) ---------- --GA-C---- -G-------- ---G----G --C------- ---------- ----------
leprae         (14) ---------- --A------- -C--T----- ---G---CTG --C--T---- -A--T----- T---------


                       648                668                688                708
tuberculosis   (1) GGCGCCAAGG AGGTCGAGAC CAAGGAGCAG ATTGCGGCCA CCGCAGCGAT TTCGGCGGGT GACCAGTCCA
bovis BCG      (2) ---------- ---------- ---------- ---------- ---------- ---------- ----------
bovis          (3) ---------- ---------- ---------- ---------- ---------- ---------- ----------
microti        (4) ---------- ---------- ---------- ---------- ---------- ---------- ----------
africanum      (5) ---------- ---------- ---------- ---------- ---------- ---------- ----------
chitae         (6) TCG------- ---------- ---------- --C--C---- ----C-G--- C--C-----C ----A-CA---
intracel. 3324 (7) TCG-----A- ---------- -----C--- --C--T---- ----G--C-- ---------C --------G-
intracellulare (8) TCG------- ---------- -----C--- --C--T---- ----G--C-- ---G----C ------CGG-
avium          (9) TCG------- ---------- ------C--- --C--T---- ----G--C-- C--C-----C --------G-
paratuberculosis (10) TCG---- ---------- ------C--- --C--T---- ----G--C-- C--C-----C --------G-
fortuitum      (11) A--------- ----G----- ---------- --C--T---- ----C-GT-- C--C--C--- ----------
malmoense      (12) TCG------- ---------- ---------- --C--T--G- ----C----- C--------C --------G-
scrofulaceum   (13) TCG------- ---------- ------C--- --C--C---- ----G----- ---------C ----- --G-
leprae         (14) -A-------- -------A-- ------A--A -----T---- -T-------- ---------- --------G-
```

Figure 1b

```
                      718                          738                 758                  778
tuberculosis (1) TCGGTGACCT GATCGCCGAG GCGATGGACA AGGTGGGCAA CGAGGGCGTC ATCACCGTCG AGGAGTCCAA
bovis BCG    (2) ---------- -
bovis        (3) ---------- -
microti      (4) ---------- -
africanum    (5) ---------- -
chitae       (6) ---------- -
intracel. 3324 ((7)---C----- -
intracellulare (8)
avium          (9) ----C----- -
paratuberculosis(10)C----- -
fortuitum (11)   ---------- ---------- --C------- ----
malmoense (12)   ----C----- -
scrofulaceum (13)----C----- -
leprae       (14) --------T-- ---------- ---------- ----
```

Figure 1c

Office européen
des brevets

**RAPPORT DE RECHERCHE EUROPEENNE**

Numero de la demande

EP 93 42 0339

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| X | WO-A-90 12875 (INSERM & INSTITUT PASTEUR)<br><br>* tout le document, et tout particulièrement pages 16-17, formules XV,XVII,XIX et XX * | 1-5,8,9,<br>11,12,<br>14,15,19 | C12N15/31<br>C12Q1/68<br>A61K39/04 |
| Y<br>D | * page 15, ligne 27 - ligne 34 *<br>& FR-A-2 645 878<br>--- | 15-19 | |
| Y<br><br>D | WO-A-91 19812 (BIO MERIEUX)<br>* page 4 - page 13 *<br>* revendications 1-19 *<br>& FR-A-2 663 040<br>--- | 15-19 | |
| D,A | WO-A-88 06591 (SCRIPPS CLINIC AND RESEARCH FOUNDATION)<br>* le document en entier, et particulièrement les peptides 15-25, page 60 *<br>--- | 13,14 | |
| D,A | JOURNAL OF BACTERIOLOGY<br>vol. 169, no. 3 , Mars 1987 , AMERICAN SOCIETY FOR MICROBIOLOGY<br>pages 1080 - 1088<br>SHINNICK, T.M. 'The 65-kilodalton antigen of Mycobacterium tuberculosis'<br>* le document en entier *<br>----- | | **DOMAINES TECHNIQUES RECHERCHES (Int.Cl.5)**<br><br>C07K<br>C12Q<br>C12N<br>A61K |

Le présent rapport a été établi pour toutes les revendications

| Lieu de la recherche | Date d'achèvement de la recherche | Examinateur |
|---|---|---|
| LA HAYE | 12 Novembre 1993 | ANDRES, S |